# EUROPEAN PATENT APPLICATION

(11) **EP 3 070 628 A1**
(43) Date of publication of application: **21.09.2016**
(21) Application number: 16161214.8
(22) Date of filing: 18.03.2016
(51) Int. Cl.: G06F 19/00

(54) **METHODS AND DEVICES FOR TRACKING PATIENT DATA**

(30) Priority: 19.03.2015 US 201562135536 P; 15.03.2016 US 201615070286
(71) Applicant: Covidien LP, Mansfield, MA 02048 (US)
(72) Inventor: MAHONEY, Patrick M., Wallingford, CT Connecticut 06492 (US)
(74) Representative: Pratt, Richard Wilson

(57) **Abstract**

Disclosed are systems, devices, and methods for tracking patient health. An exemplary method comprises storing patient data in a database accessible to one or more of a patient, a clinician, and an administrator, receiving patient health data, updating, in the database, a health record associated with the patient based on the received patient health data, determining a potential diagnosis based on the patient health data, sending the patient health data and the potential diagnosis to a clinician device, and sending information including treatment options to the clinician device based on the potential diagnosis.

## Description

### BACKGROUND

### Technical Field

The present disclosure relates to patient monitoring and, more specifically, to systems, devices, and methods for tracking patient health by monitoring patient activity levels and related health parameters.

### Description of Related Art

There is in the art a variety of patient data collection systems. For most patients entering a treatment process, the first step, before they even see a doctor or other treatment provider, is to fill out a patient questionnaire about their medical history. Systems have been developed to collect data provided in patient questionnaires into an electronic medical record (EMR) which can be shared among treatment providers such that patients are not required to fill out the same information repeatedly.

Treatment providers may also add information to the EMR, such as diagnoses, conditions, treatment, and other data collected while the patient is in the care of the treatment provider. For example, the treatment provider may question the patients about their medical history and other related factors, and may add the data collected in the EMR. However, the majority of information collected from patients about events and activities performed outside of the treatment provider's care is provided by patients relying on their memory and best estimates, which are notoriously inaccurate and leads to unreliable data.

### SUMMARY

Provided in accordance with the present disclosure is a method of tracking patient health. In an aspect of the present disclosure, the method includes storing patient data in a database accessible to one or more of a patient, a clinician, and an administrator, receiving patient health data, updating, in the database, a health record associated with the patient based on the received patient health data, determining a potential diagnosis based on the patient health data, sending the patient health data and the potential diagnosis to a clinician device, and sending information including treatment options to the clinician device based on the potential diagnosis.

In another aspect of the present disclosure, the patient health data includes risk factors including one or more of age, history of smoking, heart disease, respiratory disease, obesity, and diabetes.

In a further aspect of the present disclosure, the patient health data includes information regarding the patient's geographic location, and wherein determining the potential diagnosis includes comparing the patient's health data with data regarding known health concerns in the patient's geographic location.

In another aspect of the present disclosure, the patient health data includes one or more of a measure of the patient's activity level, nutritional information regarding food the patient consumes, sleep pattern information, heart rate information, blood pressure information, and respiratory rate information.

In a further aspect of the present disclosure, the patient health data may be viewed by the clinician in real-time.

In another aspect of the present disclosure, the patient health data is received from a monitoring device such as an activity tracker.

In a further aspect of the present disclosure, the patient health data is received from the patient device on which the patient manually enters health data.

In another aspect of the present disclosure, the method further includes sending the potential diagnosis to a patient device.

In a further aspect of the present disclosure, the method further includes determining whether the patient health data indicates a health concern, and sending an alert to a patient device when it is determined that the patient health data indicates a health concern.

In yet a further aspect of the present disclosure, the method further includes determining whether the patient is associated with a treatment provider for the alert, and sending information regarding potential treatment providers to the patient device when it is determined that the patient is not associated with a treatment provider for the alert.

In another aspect of the present disclosure, the method further includes determining whether the patient has consented to sharing the patient health data with the clinician, and sharing the patient health data with the clinician when it is determined that the patient has consented to sharing the patient data with the clinician.

Provided in accordance with the present disclosure is a non-transitory computer-readable storage medium storing instructions which, when executed by a processor, cause a computer to store patient data in a database accessible to one or more of a patient, a clinician, and an administrator, receive patient health data, update, in the database, a health record associated with the patient based on the received patient health data, determine a potential diagnosis based on the patient health data, send the patient health data and the potential diagnosis to a clinician device, send information including treatment options to the clinician device based on the potential diagnosis.

In another aspect of the present disclosure, the patient health data includes risk factors including one or more of age, history of smoking, heart disease, respiratory disease, obesity, and diabetes.

In a further aspect of the present disclosure, the patient health data includes information regarding the patient's geographic location, and wherein determining the potential diagnosis includes comparing the patient's health data with data regarding known health concerns in the patient's geographic location.

In another aspect of the present disclosure, the patient health data includes one or more of a measure of the patient's activity level, nutritional information regarding food the patient consumes, sleep pattern information, heart rate information, blood pressure information, and respiratory rate information.

In a further aspect of the present disclosure, the patient health data is received from a monitoring device such as an activity tracker.

In another aspect of the present disclosure, the instructions further cause the computer to send the potential diagnosis to a patient device.

In a further aspect of the present disclosure, the instructions further cause the computer to determine whether the patient health data indicates a health concern, and send an alert to a patient device when it is determined that the patient health data indicates a health concern.

In another aspect of the present disclosure, the instructions further cause the computer to determine whether the patient is associated with a treatment provider for the alert, and send information regarding potential treatment providers to the patient device when it is determined that the patient is not associated with a treatment provider for the alert.

In a further aspect of the present disclosure, the instructions further cause the computer to determine whether the patient has consented to sharing the patient health data with the clinician, and share the patient health data with the clinician when it is determined that the patient has consented to sharing the patient data with the clinician.

Any of the above aspects and embodiments of the present disclosure may be combined without departing from the scope of the present disclosure.

### BRIEF DESCRIPTION OF THE DRAWINGS

Various aspects and features of the present disclosure are described hereinbelow with references to the drawings, wherein:
FIG. 1 is a simplified box diagram of a hardware and software system for tracking patient health, in accordance with an embodiment of the present disclosure;
FIG. 2 is a diagram of the various devices and entities which may form part of or interact with the system of FIG. 1, in accordance with an embodiment of the present disclosure;
FIG. 3 is a flowchart of an example method for tracking patient health, in accordance with an embodiment of the present disclosure.

### DETAILED DESCRIPTION

Provided in accordance with the present disclosure are devices, systems, and methods for tracking patient health, analyzing data collected, and providing data, alerts, diagnoses, and/or treatment options to patients and treatment providers. Examples of such devices, systems incorporating such devices, and methods using the same are described below. The devices, systems, and methods disclosed herein may be applicable to providing care to patients first entering a treatment program, patients requiring acute treatment, and/or patients with chronic illness or conditions which require life-long management of disease.

The disclosed system for tracking patient health may be used by patients to individually monitor and manage their own health, and/or by patients in conjunction with treatment providers to monitor and treat medical conditions. In addition, the disclosed system for tracking patient health may analyze collected data and provide treatment providers with potential diagnoses and treatment options during treatment of a monitored patient. The system includes one or more tracking devices usable by a patient to collect data regarding the patient's health, lifestyle habits, and activities. The system further includes one or more software applications that provide the patient with information regarding the data collected, and one or more software applications that assist a treatment provider with the diagnosis, planning, and treatment of the patient. The system also includes a data management database which anonymously aggregates the treatment data from multiple patients to allow tracking of the overall treatment statistics and information of a population group over time.

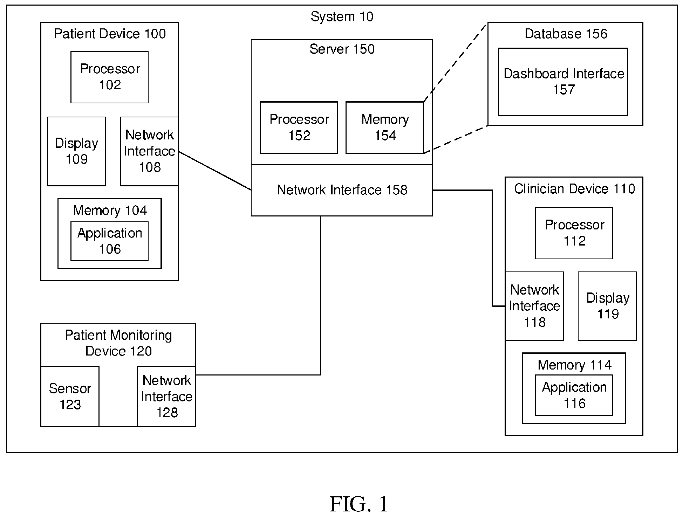

With reference to FIG. 1, a system for tracking patient health includes a patient device 100, a clinician device 110, a patient monitoring device 120, and a server 150. In some embodiments, the system may include a server which is located off-site, such as, for example, a cloud based server. Patient device 100 may be any electronic device on which a patient software application 106 may be installed, examples of which include a personal computer (PC), personal digital assistant (PDA), smartphone, laptop, tablet, and/or a wearable computer such as a smartwatch, etc. Patient device 100 includes a processor 102, a memory 104, a patient application 106 stored in memory 104 and executable by processor 102, a network interface 108, and a display 109. Display 109 may be touch sensitive and/or voice activated, enabling display 109 to serve as both an input and an output device.

Memory 104 includes any non-transitory computer-readable storage media for storing data and/or software that is executable by processor 102 and which controls the operation of the patient device 100. In an embodiment, the memory 104 may include one or more solid-state storage devices such as flash memory chips. Alternatively or in addition to the one or more solid-state storage devices, memory 104 may include one or more mass storage devices connected to the processor 102 through a mass storage controller (not shown) and a communications bus (not shown). Although the description of computer-readable media contained herein refers to a solid-state storage, it should be appreciated by those skilled in the art that computer-readable storage media can be any available media that can be accessed by the processor 102. That is, computer readable storage media includes non-transitory, volatile and nonvolatile, removable and non-removable media implemented in any method or technology for storage of information such as computer-readable instructions, data structures, program modules, or other data. For example, computer-readable storage media includes RAM, ROM, EPROM, EEPROM, flash memory or other solid state memory technology, CD-ROM, DVD, Blu-Ray or other optical storage, magnetic cassettes, magnetic tape, magnetic disk storage or other magnetic storage devices, or any other medium which can be used to store the desired information and which can be accessed by patient device 100.

Clinician device 110 is similar to patient device 100 and may be any of the electronic devices described above. Clinician device 110 includes a processor 112, a memory 114, a clinician application 116 stored in memory 114 and executable by processor 102, a network interface 118, and a display 119.

Patient monitoring device 120 may include any suitable device(s) for visual monitoring, audible monitoring, monitoring of physical characteristics, physiological conditions, other measurable characteristics or conditions, etc. For example, patient monitoring device 120 may be a temperature sensor, a heart rate monitor, ECG monitor, blood pressure monitor, pulse oximeter, pedometer, activity tracker, or any other device commonly used to monitor a patient's condition, biometrics, and/or physical activities, such as the patient's sleep cycle, the patient's water intake and/or other nutritional habits, etc. Patient monitoring device 120 may be wearable devices, such as an activity tracking device, a smart watch, a smart phone, etc., devices attachable to the patient's body, and/or implantable devices for monitoring the patient's health. One example of such a patient monitoring device 120 is the ZEPHYR^{™} Performance Systems sold by MEDTRONIC PLC.

Server 150 may be any server or collection of servers configured to collect and store data, including health data as described below, associated with various patients. Server 150 may be located at the clinician's office, at a treatment center or hospital where the patient is treated, and/or at an off-site location, e.g. a cloud-based server. Server 150 includes a processor 152, a memory 154, and a network interface 158. Memory 154 of server 150 includes a database 156 that stores data relating to the patients that are received from patient devices 100, clinician devices 110, patient monitoring devices 120, or from other sources. The data may be stored in various forms, including an electronic medical record (EMR), a screening database, and emergency room database, etc., for each patient and in some embodiments only certain of the data is available for review depending on the source of the inquiry. In one embodiment, database 156 operates as a central collection database for all patient data originating from any source, and database 156 receives data from various other databases, such as a patient activity tracking database storing data relating to a patient's activity level, a patient nutrition database storing data relating to a patient's nutritional habits, patient treatment database storing patient treatment data, a radiology database storing patient image data, a pharmacological database storing data regarding medication, and any other relevant database.

The data stored in database 156 may be standardized to interface with various other databases. For example, the data may be stored in a standardized data type used by the various other databases, and/or the data may be converted into a standardized data type when received from the various other databases and may be converted back to the native data type when sent out to other databases. The data stored in database 156 may be aggregated and analyzed by processor 152 and may be output for display to the patient and/or treatment providers through a dashboard interface 157. Dashboard interface 157 is the visible portion of an application stored in memory 154 and is executable by processor 152 to provide the patient and/or treatment providers with statistics and data regarding the patient's health, potential diagnosis and/or risk factors, and treatment options and outcomes or other similar data for later analysis. Risk factors may include the patient's age, a history of smoking, heart disease, respiratory disease, obesity, and/or diabetes, among others. Risk factors may also include previous diagnoses. For example, a patient who has been diagnosed as suffering from metabolic syndrome may present a number of different risk factors, including high blood pressure, high blood sugar, excess body fat around the waist, and/or abnormal cholesterol levels. These conditions, when occurring together, may increase the patient's risk of developing heart disease and diabetes, and/or having a stroke. In at least one embodiment, the data stored in database 156 is anonymized and aggregated to protect patient privacy.

Patient device 100, clinician device 110, and patient monitoring device 120 are each connected to network interface 158 of server 150 via their respective network interfaces 108, 118, and 128. For example, network interfaces 108, 118, 128, and 158 may be connected through a network such as a local area network (LAN) consisting of a wired network and/or a wireless network, a wide area network (WAN), a wireless mobile network, a Bluetooth network, and/or the internet. Patient device 100 and patient monitoring device 120 may further include a radio frequency identification (RFID) and/or near-field communication (NFC) capability.

Patient device 100 and patient monitoring device 120 are configured to provide patient health data to and access patient health data from server 150. The patient health data may include any data relating to the patient's health including profile data, activity data, nutrition data, weight measures, glucose levels, sleep data, temperature, medical records, pharmaceutical records, geographic location information, etc. The profile data may include the patient's age, height, weight, profession, family history, etc. The activity data may include metrics recorded by activity tracking devices, such as steps walked, workouts performed, estimated calories burned, heart rate measures, respiratory rate measures, etc. Nutrition data may include information as to the patient's food intake, such as types of food, weights of food, composition of food, estimated caloric content of food, etc. The medical records may include the patient's medical history, current and previous diagnoses and illnesses treated or managed, surgeries or other treatments received, allergies, risk factors, etc. The pharmaceutical records may include which medications the patient is currently prescribed, the dosages of such medications, as well as information on which medications the patient has previously taken and medications to which the patient may have experienced adverse reactions. The geographic information may include where the patient lives, works, and frequently visits, for example, if the patient lives or works in a city with known air or water quality problems and/or other environmental concerns, and travel information, for example, if the patient travels to an area where a particular disease is common.

Server 150 may compile the patient health data into a health record, such as an electronic medical record (EMR). Server 150 may also receive patient health data from other sources, as described below, and add such patient health data to the patient's health record. The patient's health record may additionally be stored on patient device 100 and/or patient monitoring device 120. In an embodiment, patient device 100 and/or patient monitoring device 120 may include an emergency authorization to give an emergency care provider access to the patient health data and/or patient health record, either directly from patient device 100 and/or patient monitoring device 120, or via a link to server 150. For example, in a scenario where the patient is unconscious or otherwise incapable of giving consent while in need of emergency treatment, the emergency authorization included in patient device 100 and/or patient monitoring device 120 may be detected, such as by scanning patient device 100 and/or patient monitoring device 120 for the RFID and/or NFC capability to receive the emergency authorization.

Server 150 may further use the patient health data and/or patient health records of a population of patients to compile treatment statistics and models for patients who have similar health, activity, treatment, and/or geographic profiles. For example, general population health trends may be determined based on health and activity data collected from a population of patients who have similar activity and nutrition habits. Additionally, when a number of patients in a monitored population group develop similar diseases, server 150 may issue a general health alert to hospitals, other treatment providers, and health authorities in a particular area. Server 150 may also determine whether patient health data collected for a particular patient conforms to general trends. For example, if conflicting patient health data is received, such as activity data reflecting a high activity level combined with normal nutrition intake, but significant increases in the patient's glucose levels and/or weight, an alert may be provided to the patient and/or a clinician treating the patient.

While patient application 106 and clinician application 116 are individually illustrated in Fig. 1 and described as separate applications herein, these applications may form part of a single unified application that can be configured to operate differently based on the user, i.e. a patient or clinician. Clinician application 116 may be specially configured for various clinicians depending their particular needs. Alternatively, patient application 106 and clinician application 116 may be stand-alone applications operating separately from each other. Additionally, patient application 106 and clinician application 116 may in turn be broken up into various sub-applications specially configured for a particular purpose, i.e. activity tracking, nutrition tracking, risk factors, diagnoses, treatment options, outcomes, etc.

Clinician application 116 may be configured to interface with various other applications used by clinicians during diagnosis and treatment of patients. For example, clinician application 116 may receive data inputted via other applications used by the clinician, and/or send data to the other applications.

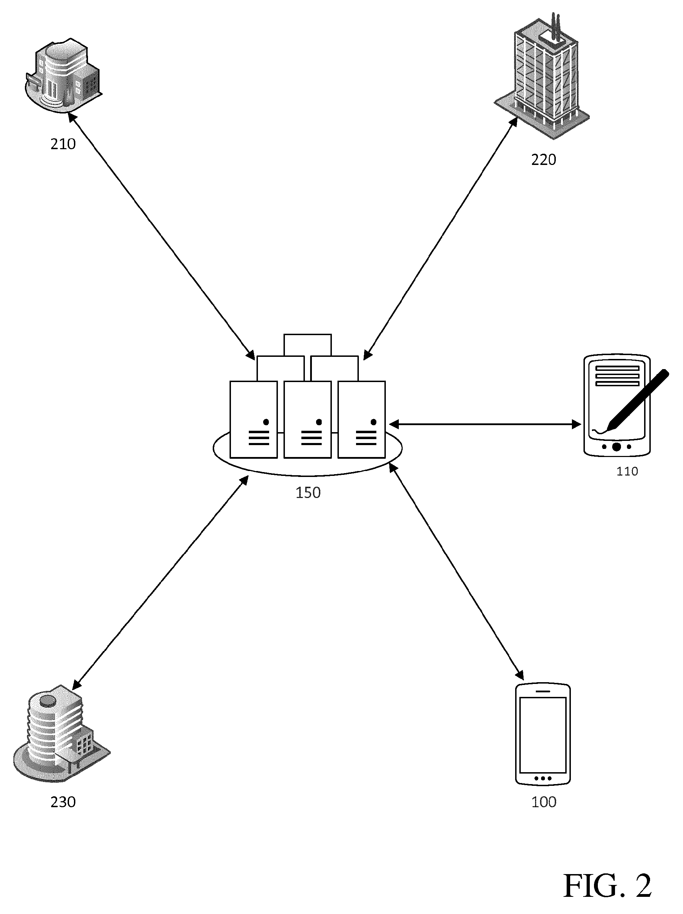

Turning now to FIG. 2, there is shown a diagram of various devices and entities which may interact with server 150 of FIG. 1. In addition to patient device 100 and clinician device 110, described above with reference to FIG. 1, a hospital 210 may also receive patient data from and send patient data directly to server 150. The clinician using clinician device 110 may be associated with hospital 210 or may operate separately from hospital 210. Similarly, an insurer 220 may also receive patient data from and send patient data directly to server 150. Insurer 220 may, for example, be a health insurance agency or network or governmental agency such as Medicare with which the patient is associated. Additionally, a health authority 230 may receive patient data from and send patient data directly to server 150. Health authority 230 may be, for example, a city or state health authority, a national health authority such as the United States Centers for Disease Control and Prevention (CDC), and/or an international organization such as the World Health Organization (WHO).

Depending on the laws and regulations of a particular jurisdiction in which system 100 is to operate, one or more forms of consent and/or authorization may be required for patient data to be shared amongst the various entities described above. In such embodiments, system 100 may be restricted to share patient-specific data with only the entities for which the patient has consented and authorized data to be shared. Anonymized patient data and general analytics and statistics regarding patient data may also be shared among the various devices and entities of system 100 where permitted.

Hospital 210 may be associated with server 150 in a way that hospital 210 is capable of receiving patient health records from server 150. Hospitals that are associated with server 150 may be preferred by patients who have consented to having their patient health data collected by server 150 because such hospitals may be better able to treat those patients for whom patient health records are maintained by server 150. Additionally or alternatively, these hospitals may offer a variety of incentives and programs to the patients in exchange for making their health data available such as price reductions or streamlined appointment procedures, and improved or enriched care engagement. In some instances, insurer 220 may mandate that the patient make their data available to in-network hospitals 210 as a requirement for coverage. In one embodiment, this data regarding insured patients is used to provide ever more accurate and even individualized pricing and risk assessment. Further, this data can be used to provide enticements and motivations to patients for modifying their behavior. For example, by reviewing activity levels an insurance company can offer a price incentive to an individual who demonstrates, via the data, a commitment to increased activity levels, such as walking two miles three to four times per week. This type of change in behavior is beneficial to the patient from a health standpoint, beneficial to the insurance company in likely reduced risk of certain diseases, and therefore the enticement of a reduced premium for demonstrating this activity may lead to actual changes in behavior. A variety of other behavioral modifications and enticements for such modifications can be generated from the patient data and provided to the patient in an effort to improve the patient's health as well as reduce the costs and burdens on the medical systems associated with not addressing these behavioral issues.

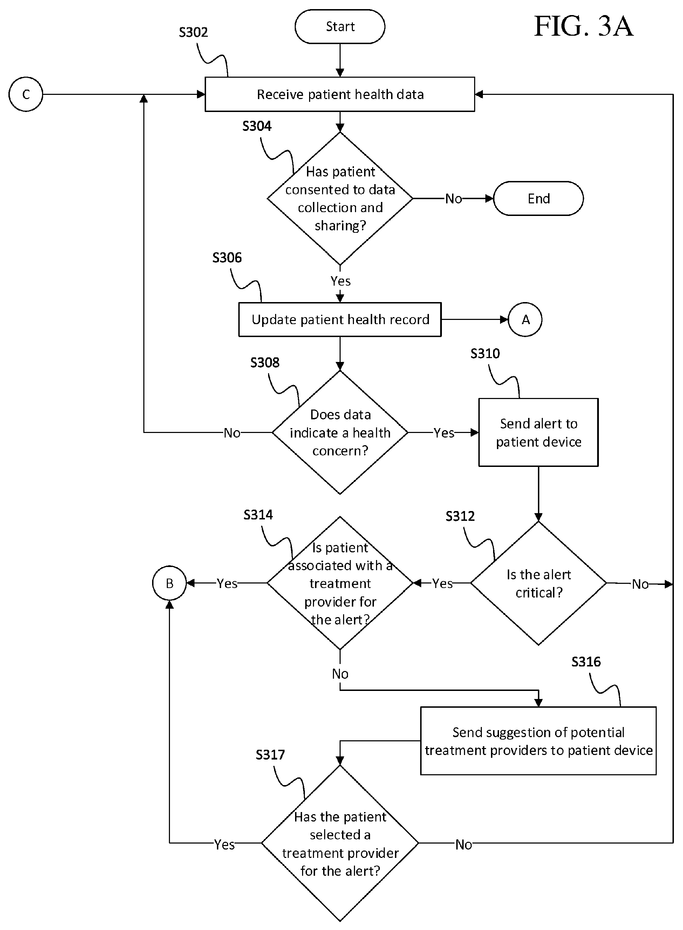

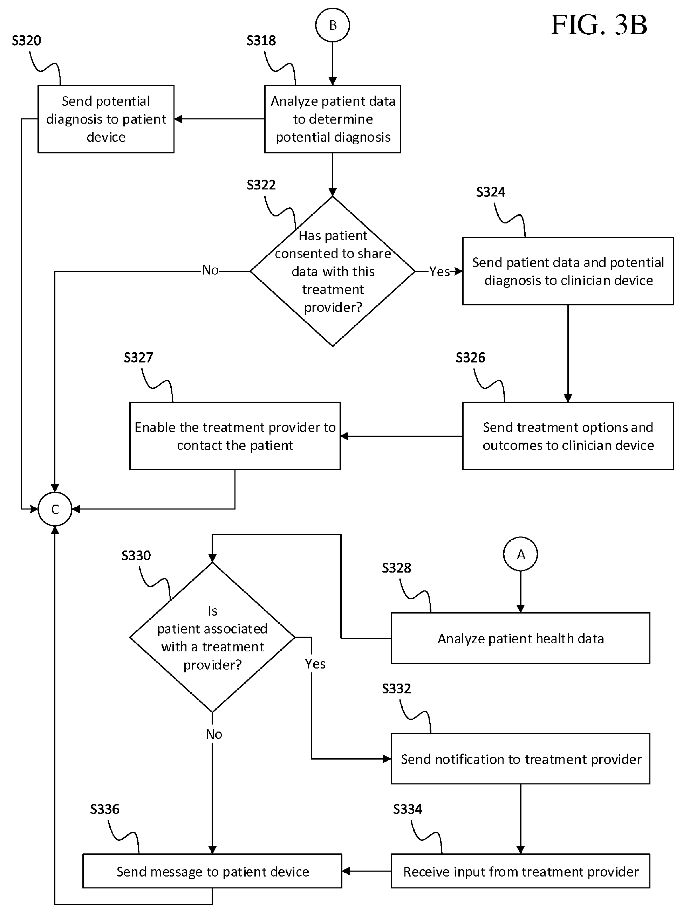

With reference to FIG. 3, there is shown a flowchart of an exemplary method of tracking patient health, according to an embodiment of the present disclosure. Starting at step S302, patient device 100 or patient monitoring device 120 collects patient health data. Such devices may include wearable computing devices such as activity trackers, smartwatches, and the like; other computing devices such as smartphones, tablet computers, laptop computers, desktop computers, and the like, by means of which a user may manually enter data, or which may collect data from the wearable computing devices. Application 106 running on patient device 100 and/or patient monitoring device 120 may include an indication from the patient that the patient health data is to be provided to server 150. If authorized, server 150 may also receive the patient health data from other sources, such as other servers, hospital computers, clinician device 110, and/or any of the other devices mentioned above.

After collecting patient health data locally on the patient device 100 or patient monitoring device 120, in a communication (which may be automated) a determination may be made at step S304 as to whether the patient has consented to data collection by server 150, or whether the patient would prefer to retain such information for private uses. A further determination may be made as to whether the patient has consented to the collected data being shared with third parties, such as treatment providers, hospitals, etc. If the patient has not consented to data collection, the method ends. However, if the patient has consented to data collection, the method proceeds to step S306, where a patient health record is created and/or updated. For example, an electronic medical record (EMR) for the patient may be created and/or retrieved from another source. If the patient consents to data collection by server 150 but not to data sharing with third parties, some of the features of the present disclosure may not be available. In an embodiment, when it is determined that the patient has not consented to data collection, server 150 may send a message to the patient to notify the patient that data will not be collected unless the patient consents to data collection, and give the patient an opportunity to consent to data collection. After updating the patient health record, the method divides into two branches, the first branch continuing at step S308, and the second branch continuing at step S328.

At step S308, a determination may be made as to whether the received patient health data indicates a health concern or requires comment. For example, if the patient is undergoing treatment for obesity, metabolic syndrome, or other chronic conditions and patient health data is received which indicates that the patient is overeating and/or has not performed sufficient physical activity, such patient health data may indicate a health concern. In another example, if the patient is suffering from diabetes, and an abnormal increase or drop in glucose levels is detected, such patient health data may also indicate a health concern. As those skilled in the art will recognize, there may be an infinite number of additional examples of patient health data being indicative of a health concern. However, for purposes of brevity, every possible example will not be described here. If it is determined that the received patient health data indicates a health concern or necessitates other comment, the method proceeds to step S310, where an alert is sent to patient device 100 regarding the health concern or including the comment. If it is determined that a health concern is not indicated by the health data or that no comment is necessary, the method returns to step S302.

Further, the alert sent to patient device 100 may be in the form of a reminder to perform physical activity, log nutrition information, take medication, take a blood pressure or glucose level measurement, etc. In another example, the patient health data may indicate a crucial health concern, such as a severe increase or drop in blood pressure, cardiac activity, respiration rate, etc. In such scenarios, a critical alert may be sent to patient device 100 to notify the patient to seek urgent treatment.

Therefore, at step S312, a determination is made whether the alert is related to a critical health concern. If the alert is not related to a critical health concern, the alert is displayed to the user without further action, and the method returns to step S302. However, if the alert is related to a critical health concern, the method proceeds to step S314. In some embodiments, all alerts, including alerts determined not to be related to a critical health concern, are logged, and patient activity subsequent to an alert may be compared with regular patient activity. For example, if patient device 100 and/or patient monitoring device 120 determine that the patient has reached a daily caloric intake condition, a non-critical alert may be displayed to notify the patient that the daily caloric intake condition has been reached and that the patient should not consume any more food that day. If the patient continues to consume food after the alert is displayed, weight-loss progress may suffer and changes in the patient's behavior may be needed, and counseling from a treatment provider may be needed. In another example, if patient device 100 and/or patient monitoring device 120 determines that the patient has not met a daily activity goal for a number of days, an alert may be displayed informing the patient that additional daily activity may be required in order to reach weight-loss goals, and the patient's activity level after the alert is presented may be monitored to determine if the patient is reacting to the alert or not.

At step S314, a determination is made as to whether the patient is associated with a treatment provider for the alert. If the patient is not associated with a treatment provider for the alert, the method proceeds to step S316, where a message including suggested treatment providers is sent to patient device 100. The application may provide the ability for the patient to both contact the treatment provider and grant them access to the patient's health data. If such options are selected by the patient in step S317, the method proceeds to step S318, as if the patient had already been associated with the treatment provider. In accordance with one embodiment of the present disclosure, because the treatment provider is associated with the system for tracking patient health, they have already consented to being contacted by patients in this manner, even if no prior relationship exists. Other more traditional scenarios are also contemplated within the scope of this disclosure, where only following being contacted by the patient and scheduling an appointment is the patient health data made available, thus giving the treatment provider the option of declining the new patient.

Alternatively, if the patient is already associated with a treatment provider for the alert, the method proceeds to step S318, where the patient health data is analyzed to determine a potential diagnosis, such as a computer-aided diagnosis (CAD). For example, if the patient is being treated for heart disease and the alert is related to abnormal cardiac activity, the patient health data may be analyzed to determine whether the patient is having a heart attack or is simply performing strenuous physical activity.

Thereafter, at step S320, the potential CAD diagnosis is sent to patient device 100. For example, if the patient health data indicates that the patient is having a heart attack, an alert may be sent to patient device 100 warning the patient of the potential CAD diagnosis of a heart attack, and informing the patient to either contact emergency care, and/or that help has already been summoned. Alternatively, if the alert is triggered by strenuous physical activity, a cautionary communication can be sent alerting the patient to the potential dangers and providing them with an immediate protocol to follow to bring the patient back within their normal health parameters. For example, a diabetic patient could be warned of the need to immediately ingest certain types of food or inject insulin to bring blood sugar levels within the normal range for that patient.

Concurrently with step S320, a determination is made, at step S322, as to whether the patient has consented to the patient's health data being shared with a particular treatment provider, i.e., the treatment provider with which the patient is associated for the alert. If the patient has not consented to sharing data with the treatment provider, the method proceeds to step S302. However, if the patient has consented to sharing data with the treatment provider, the patient health data and potential CAD diagnosis is sent to clinician device 110 at step S324. Thereafter, or concurrently therewith, at step S326, potential treatment options and/or outcomes of those treatments with similar patients is sent to clinician device 110.

After reviewing the patient health data and potential CAD diagnosis, the treatment provider may decide to contact the patient. Thus, at step S327, application 116 executing on clinician device 110 enables the treatment provider to contact the patient. For example, application 116 may enable the treatment provider to contact the patient directly through application 116, such as by initiating a telephone call and/or sending an instant or electronic mail message to the patient, or by providing the patient's contact information to the treatment provider such that the treatment provider may contact the patient via other means.

As noted above, after step S306, the method divides into two branches, with the second branch continuing to step S328. Thus, after updating the patient health record at step S306, the patient health record is analyzed, at step S328, for example to determine whether the patient health record indicates whether the patient is complying with a treatment plan. This analysis is part of routine tracking and monitoring of activity and health parameters of a patient. For example, as part of a treatment plan for a patient, various conditions and goals, such as a daily caloric intake condition, a daily activity goal, a daily step count goal, etc., may be configured. The treatment plan may be configured and/or adjusted by a treatment provider, and/or may be created and/or adjusted by the patient. The patient health data may be analyzed to determine whether the patient is complying with the treatment plan such as by meeting the conditions and goals. This data provides a mechanism to check on the patient's self-reporting of activities which is notoriously poor. As with all endeavors, the better the data, the better the analysis and in health concerns the better the prescription for treatment, etc.

Next, at step S330, a determination is made as to whether the patient is associated with a treatment provider. For example, the patient may be associated with a primary care physician and/or a specialized treatment provider, such as, in the example used above, a bariatric weight-loss treatment provider. Thus, in at least one embodiment, the treatment provider may review the patient's health data in real time after being notified that the patient's health record has been updated.

If the patient is not associated with a treatment provider, the method proceeds to step S336. However, if the patient is associated with a treatment provider, the method proceeds to step S332, where a notification may be sent to one or more of the treatment providers with whom the patient is associated to notify the treatment providers of the update to the patient's health record and of the analysis performed at step S328. Thereafter, the treatment provider may review the patient's health record and provide additional analysis of the patient's health data, which may be received by server 150 at step S334.

At step S336, a message is sent to patient device 100 and/or patient monitoring device 120 informing the patient of the analysis performed at step S328 and/or the treatment provider's comments received at step S334. For example, the treatment provider may provide a comment of encouragement to the patient in view of the collected patient health data. This may be the case where the patient health data reveals that the bariatric patient's food intake was within the prescribed limits and/or that the patient undertook sufficient activity such as walking two miles, and therefore a note of encouragement is appropriate. Such positive affirmations have proven helpful encouragement for bariatric patients, both pre- and post-surgery. A variety of additional comments may also be provided as deemed appropriate by the treatment provider in the treatment of a particular disease. In another example, the treatment provider, upon reviewing the patient health data, may determine that the patient would benefit from a meeting, and thus a message may be sent to patient device 100 and/or patient monitoring device 120 suggesting that the patient make an appointment for a visit with the treatment provider. After sending the message to patient device 100 and/or patient monitoring device 120, the method returns to step S302.

While all of the above-described steps are occurring, data from each patient using patient device 110 is collected by server 150 in database 156 (FIG. 1). The data is aggregated and analyzed by processor 152 on server 150 and presented to medical personnel via dashboard interface 157. The data may also be anonymized. Dashboard interface 157, allows medical personnel, for example, administrators, clinicians, staff at a hospital, treatment center, other medical facility, insurance provider, or government agency to monitor and correlate patient outcomes to clinical processes, compare against national and best-in-class practices, and drive data-driven management. Dashboard interface 157 may present aggregated and analyzed data anonymously to protect patient privacy.

The data of various patients are collected and anonymized. Data regarding treatments prescribed, outcomes of those treatments, and subsequent developments are stored in database 156. Statistics may be compiled or derived based on the data to provide predictive results of a particular treatment option based on the condition of a particular patient. Dashboard interface 157 may also be used to view such data and statistics to assist clinicians in diagnosing patients, prescribing treatments to patients, and determining the best course of action based on past results. That is, by inputting observed parameters of the patient, a correlation can be made to other individuals displaying the same or similar symptoms. As can be appreciated, the more granular the data input, the closer the correlation possible. With this information, a resource can be provided to clinicians and treatment providers to assist in identifying and treating patients, particularly those patients who are not responding to current treatments. Moreover, the system provides a confirmatory check on the treatment provider's diagnosis to ensure that they have not overlooked a potential health concern.

While several embodiments of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope and spirit of the claims appended hereto.

The invention may be described by reference to the following numbered paragraphs:-
1. A method of tracking patient health, the method comprising:
   storing patient data in a database accessible to one or more of a patient, a clinician, and an administrator;
   receiving patient health data;
   updating, in the database, a health record associated with the patient based on the received patient health data;
   determining a potential diagnosis based on the patient health data;
   sending the patient health data and the potential diagnosis to a clinician device; and
   sending information including treatment options to the clinician device based on the potential diagnosis.
2. The method according to paragraph 1, wherein the patient health data includes risk factors including one or more of age, history of smoking, heart disease, respiratory disease, obesity, and diabetes.
3. The method according to paragraph 1, wherein the patient health data includes information regarding the patient's geographic location, and wherein determining the potential diagnosis includes comparing the patient's health data with data regarding known health concerns in the patient's geographic location.
4. The method according to paragraph 1, wherein the patient health data includes one or more of a measure of the patient's activity level, nutritional information regarding food the patient consumes, sleep pattern information, heart rate information, blood pressure information, and respiratory rate information.
5. The method according to paragraph 1, wherein the patient health data may be viewed by the clinician in real-time.
6. The method according to paragraph 1, wherein the patient health data is received from a monitoring device such as an activity tracker.
7. The method according to paragraph 1, wherein the patient health data is received from the patient device on which the patient manually enters health data.
8. The method according to paragraph 1, further comprising sending the potential diagnosis to a patient device.
9. The method according to paragraph 1, further comprising:
   determining whether the patient health data indicates a health concern; and
   sending an alert to a patient device when it is determined that the patient health data indicates a health concern.
10. The method according to paragraph 9, further comprising:
   determining whether the patient is associated with a treatment provider for the alert; and
   sending information regarding potential treatment providers to the patient device when it is determined that the patient is not associated with a treatment provider for the alert.
11. The method according to paragraph 1, further comprising:
   determining whether the patient has consented to sharing the patient health data with the clinician; and

   sharing the patient health data with the clinician when it is determined that the patient has consented to sharing the patient data with the clinician.
12. A non-transitory computer-readable storage medium storing instructions which, when executed by a processor, cause a computer to:
   store patient data in a database accessible to one or more of a patient, a clinician, and an administrator;
   receive patient health data;
   update, in the database, a health record associated with the patient based on the received patient health data;
   determine a potential diagnosis based on the patient health data;
   send the patient health data and the potential diagnosis to a clinician device;
   send information including treatment options to the clinician device based on the potential diagnosis.
13. The non-transitory computer-readable medium according to paragraph 12, wherein the patient health data includes risk factors including one or more of age, history of smoking, heart disease, respiratory disease, obesity, and diabetes.
14. The non-transitory computer-readable medium according to paragraph 12, wherein the patient health data includes information regarding the patient's geographic location, and wherein determining the potential diagnosis includes comparing the patient's health data with data regarding known health concerns in the patient's geographic location.
15. The non-transitory computer-readable medium according to paragraph 12, wherein the patient health data includes one or more of a measure of the patient's activity level, nutritional information regarding food the patient consumes, sleep pattern information, heart rate information, blood pressure information, and respiratory rate information.
16. The non-transitory computer-readable medium according to paragraph 12, wherein the patient health data is received from a monitoring device such as an activity tracker.
17. The non-transitory computer-readable medium according to paragraph 12, wherein the instructions further cause the computer to send the potential diagnosis to a patient device.
18. The non-transitory computer-readable medium according to paragraph 12, wherein the instructions further cause the computer to:
   determine whether the patient health data indicates a health concern; and
   send an alert to a patient device when it is determined that the patient health data indicates a health concern.
19. The non-transitory computer-readable medium according to paragraph 12, wherein the instructions further cause the computer to:
   determine whether the patient is associated with a treatment provider for the alert; and

   send information regarding potential treatment providers to the patient device when it is determined that the patient is not associated with a treatment provider for the alert.
20. The non-transitory computer-readable medium according to paragraph 12, wherein the instructions further cause the computer to:
   determine whether the patient has consented to sharing the patient health data with the clinician; and
   share the patient health data with the clinician when it is determined that the patient has consented to sharing the patient data with the clinician.

## Claims

1. A method of tracking patient health, the method comprising:
storing patient data in a database accessible to one or more of a patient, a clinician, and an administrator;
receiving patient health data;
updating, in the database, a health record associated with the patient based on the received patient health data;
determining a potential diagnosis based on the patient health data;
sending the patient health data and the potential diagnosis to a clinician device; and
sending information including treatment options to the clinician device based on the potential diagnosis.

2. The method according to claim 1, wherein the patient health data includes risk factors including one or more of age, history of smoking, heart disease, respiratory disease, obesity, and diabetes and/or wherein the patient health data includes information regarding the patient's geographic location, and wherein determining the potential diagnosis includes comparing the patient's health data with data regarding known health concerns in the patient's geographic location and /or wherein the patient health data includes one or more of a measure of the patient's activity level, nutritional information regarding food the patient consumes, sleep pattern information, heart rate information, blood pressure information, and respiratory rate information.

3. The method according to claim 1, wherein the patient health data may be viewed by the clinician in real-time.

4. The method according to claim 1, wherein the patient health data is received from a monitoring device such as an activity tracker and/or wherein the patient health data is received from the patient device on which the patient manually enters health data.

5. The method according to claim 1, further comprising sending the potential diagnosis to a patient device.

6. The method according to claim 1, further comprising:
determining whether the patient health data indicates a health concern; and sending an alert to a patient device when it is determined that the patient health data indicates a health concern and/or further comprising:
determining whether the patient is associated with a treatment provider for the alert; and
sending information regarding potential treatment providers to the patient device when it is determined that the patient is not associated with a treatment provider for the alert.

7. The method according to claim 1, further comprising:
determining whether the patient has consented to sharing the patient health data with the clinician; and
sharing the patient health data with the clinician when it is determined that the patient has consented to sharing the patient data with the clinician.

8. A non-transitory computer-readable storage medium storing instructions which, when executed by a processor, cause a computer to:
store patient data in a database accessible to one or more of a patient, a clinician, and an administrator;
receive patient health data;
update, in the database, a health record associated with the patient based on the received patient health data;
determine a potential diagnosis based on the patient health data;
send the patient health data and the potential diagnosis to a clinician device;
send information including treatment options to the clinician device based on the potential diagnosis.

9. The non-transitory computer-readable medium according to claim 12, wherein the patient health data includes risk factors including one or more of age, history of smoking, heart disease, respiratory disease, obesity, and diabetes and/or wherein the patient health data includes information regarding the patient's geographic location, and wherein determining the potential diagnosis includes comparing the patient's health data with data regarding known health concerns in the patient's geographic location, and/or wherein the patient health data includes one or more of a measure of the patient's activity level, nutritional information regarding food the patient consumes, sleep pattern information, heart rate information, blood pressure information, and respiratory rate information.

10. The non-transitory computer-readable medium according to claim 8, wherein the patient health data is received from a monitoring device such as an activity tracker.

11. The non-transitory computer-readable medium according to claim 8, wherein the instructions further cause the computer to send the potential diagnosis to a patient device.

12. The non-transitory computer-readable medium according to claim 8, wherein the instructions further cause the computer to:
determine whether the patient health data indicates a health concern; and
send an alert to a patient device when it is determined that the patient health data indicates a health concern.

13. The non-transitory computer-readable medium according to claim 8, wherein the instructions further cause the computer to:
determine whether the patient is associated with a treatment provider for the alert; and
send information regarding potential treatment providers to the patient device when it is determined that the patient is not associated with a treatment provider for the alert.

14. The non-transitory computer-readable medium according to claim 8, wherein the instructions further cause the computer to:
determine whether the patient has consented to sharing the patient health data with the clinician; and
share the patient health data with the clinician when it is determined that the patient has consented to sharing the patient data with the clinician.

15. A system for tracking patient health, the system comprising:
a computer storing instructions which, when executed by a processor of the computer, cause a computer to
store patient data in a database accessible to one or more of a patient, a clinician, and an administrator,
receive patient health data,
update, in the database, a health record associated with the patient based on the received patient health data,
determine a potential diagnosis based on the patient health data,
send the patient health data and the potential diagnosis to a clinician device, and
send information including treatment options to the clinician device based on the potential diagnosis;
the system optionally further comprising a monitoring device such as an activity tracker configured to send the health data to the computer.
